# EUROPÄISCHE PATENTSCHRIFT

(11) **EP 2 102 352 B1**
(45) Veröffentlichungstag und Bekanntmachung des Hinweises auf die Patenterteilung: **25.05.2016**
(21) Anmeldenummer: 07848062.1
(22) Anmeldetag: 11.12.2007
(51) Int. Cl.: C12P 13/04, C12P 13/06, C12P 13/08, C12P 13/10, C12P 13/12, C12P 13/14, C12P 13/20, C12P 13/22, C12P 13/24

(54) **VERFAHREN ZUR GEWINNUNG VON PRODUKTEN, DIE VERSCHIEDENE KONZENTRATIONEN EINER FERMENTATIV HERGESTELLTEN VERBINDUNG ENTHALTEN**
METHOD FOR OBTAINING DIFFERENT CONCENTRATIONS OF PRODUCTS COMPRISING TARGET SUBSTANCES
PROCÉDÉ POUR OBTENIR DES PRODUITS CONTENANT DIFFÉRENTES CONCENTRATIONS DE SUBSTANCES CIBLES

(30) Priorität: 23.12.2006 DE 102006061479
(43) Veröffentlichungstag der Anmeldung: 23.09.2009
(73) Patentinhaber: Evonik Degussa GmbH, 45128 Essen (DE)
(72) Erfinder: MOLL, Matthias, 63755 Alzenau (DE); STOCKHAMMER, Stefan, 61130 Nidderau (DE); ALT, Hans Christian, 63571 Gelnhausen (DE); POHLISCH, Joachim, 63571 Gelnhausen (DE); HUTHMACHER, Klaus, 63571 Gelnhausen (DE)
(86) Internationale Anmeldenummer: PCT/EP2007/063738
(87) Internationale Veröffentlichungsnummer: WO 2008/077774

(56) Entgegenhaltungen:
- WO-A-2005/059155
- DE-B- 1 268 569
- DE-B- 1 293 707
- DE-C1- 3 630 878
- FR-A- 2 588 016
- US-A- 3 189 526
- US-A- 4 777 051

## Beschreibung

Die Erfindung bezieht sich auf ein Verfahren zur Gewinnung mindestens einer Zielsubstanz, ausgewählt aus L-Aminosäuren , in verschiedenen Konzentrationen enthaltenden Produkten, wobei die Zielsubstanzen durch Fermentation hergestellt wird (werden).

Tierfuttermittel werden insbesondere mit Aminosäuren oder Vitaminen entsprechend dem Bedarf der Tiere supplementiert. Zur Supplementierung von Tierfuttermitteln, z. B. mit L-Threonin, wird bisher ein kristallines L-Threonin mit einem Wirkstoffgehalt > 98 % eingesetzt. Das durch Fermentation hergestellte L-Threonin, muss zur Isolierung des reinen, kristallinen Stoffes von den übrigen Bestandteilen der rohen Fermentationsbrühe in aufwendigen Verfahrensschritten abgetrennt und zur Kristallisation gebracht werden. Dabei müssen eine große Anzahl von Nebenprodukten und die zur Aufarbeitung notwendigen Reagentien als Abfall entsorgt werden. Ein solcher Isolierprozeß ist darüber hinaus immer mit einem Ausbeuteverlust verbunden, da in den Abfallströmen ein Anteil an L-Threonin nicht zu vermeiden ist. Für die Verwendung im Tierfutter ist aber keine hohe Reinheit der Aminosäure notwendig. Zudem enthält die Fermentationsbrühe oft noch nutritiv wirksame Wertstoffe, so dass es zweckmäßig erscheint die fermentativ hergestellte Aminosäure zusammen mit den weiteren Bestandteilen der Fermentationsbrühe in ein festes Tierfuttermittel zu überführen.

Verfahren zur fermentativen Herstellung von L-Aminosäuren und zu deren Aufreinigung sind seit langem bekannt. Die Herstellung von L-Threonin wird z. B. in der US 4,278,765, die von L-Tryptophan, L-Phenylalanin und L-Tyrosin in der US 5, 605, 818 und die von L-Asparaginsäure in der DE 1268569 beschrieben. Verfahren zur Herstellung von Aminosäuren enthaltenden Tierfuttermittel-Zusätzen auf Fermentationsbrühebasis sind aus der US 4,777,051, der EP 0 615 693 B und der EP 0 533 039 B bekannt.

Die US 4,777,051 offenbart ein Sprühtrocknungsverfahren mit nachgeschaltetem zusätzlichen Trocknungsschritt. Tryptophan- oder Threonin-Lösungen unterschiedlichen Ursprungs mit einem L-Aminosäure-Gehalt von 20 - 60 wt-%, bezogen auf den Gesamt-Feststoffgehalt, werden in einem ersten Schritt zu einem halbtrockenen Granulat mit einer Restfeuchte von 5-15% versprüht. Anschließend wird das feuchte Granulat auf einem Förderbandtrockner mit Lochboden ausgebreitet und mit heißer Luft endgetrocknet, wobei ein Produkt von etwa 4 Gew-% Restfeuchte erhalten wird.

Gemäß EP 0 615 693 wird die Granulation ebenfalls in einem zweistufigen Trocknungsverfahren durchgeführt.

Die Fermentationsbrühe wird nach Entfernung eines Teils der Inhaltsstoffe zu einem Feinkorn, das zu mind. 70 Gew.-% eine maximale Partikelgröße von 100µm hat, sprühgetrocknet, und das so erhaltene Feinkorn in einer zweiten Stufe zu einem Granulat aufgebaut, das zu mind. 30 Gew.-% Feinkorn enthält.

Aus der EP 0 809 940 B1 ist ebenfalls ein Verfahren zur Granulation eines Tierfuttermittel-Zusatzes auf Fermentationsbrühe-Basis bekannt. Das Verfahren ist dadurch gekennzeichnet, dass die Fermentationsbrühe in einer Wirbelschicht in einem Schritt granuliert, kompaktiert und getrocknet wird, während eine zur Einstellung eines gewünschten Korndurchmessers und einer gewünschten Schüttdichte ausreichende Energiemenge zusätzlich zu der zur Erzeugung der stationären Wirbelschicht benötigten Energie auf mechanischem Weg in die Wirbelschicht eingetragen wird.

Diesen Verfahren ist gemeinsam, dass man
a) die die gewünschte Zielsubstanz produzierenden Mikroorganismen in einem geeigneten Medium fermentiert und die Zielsubstanz dort anreichert (accumulated),
b) anschließend die während der Fermentation gebildete Biomasse ganz oder teilweise abtrennt,
c) die so erhaltene Lösung oder Suspension durch Wasserentfernung aufkonzentriert und
d) daraus durch bekannte Maßnahmen wie z. B. Granulation, Sprühgranulation oder Aufbaugranulation ein festes diese Zielsubstanz enthaltendes Produkt gewinnt.

Der Zielsubstanzgehalt der Fermentationsbrühe wird nach Abschluß der Fermentation vorteilhaft analysiert, da aufgrund verschiedener Umstände die Ausbeuten der Fermentationsverfahren schwanken können. Diese Schwankungen werden dann entweder durch Zusatz von Zielsubstanz bei zu geringen Konzentrationen im Produkt oder Zusatz von inerten Zuschlagstoffen bei zu hoher Konzentration ausgeglichen (Standardisierung). Nachteilig wirkt sich bei diesen Verfahren der Gehalt an Fremdsubstanzen aus der Fermentation im Produkt auf die Transportkosten aus. Der Vorteil dieser Verfahren liegt darin, dass das Produkt die gesamte Menge der Zielsubstanz enthält, da kein Ausbeuteverlust durch die Abtrennung von Mutterlaugen eintritt, wie sie bei der Isolierung des reinen, auskristallisierten Zielverbindung notwendig wird.

Da diese keine Fremdanteile enthalten, fallen bei den gereinigten Zielverbindungen bezogen auf den Wirkstoffanteil geringere Transportkosten bei der Auslieferung an.

Außerdem entfällt bei der Isolierung des kristallinen Zielprodukts die oben beschriebene Notwendigkeit der Standardisierung der Konzentration in der Fermentationsbrühe.

Aufgabe der Erfindung ist die Bereitstellung eines Verfahrens, das die üblichen Ausbeuteverluste bei der Aufarbeitung zum reinen Zielprodukt weitgehend oder vollständig vermeidet und die Herstellung eines festen Produkts und eines festen Produkts mit einem geringeren Anteil an Zielsubstanz ermöglicht.

Gegenstand der Erfindung ist ein Verfahren zur Herstellung von Produkten, die mindestens eine fermentativ hergestellte Zielsubstanz enthalten in mehreren Verfahrensstufen, bei denen man
a) die die gewünschte Zielsubstanz produzierenden Mikroorganismen in einem geeigneten Medium fermentiert und die Zielsubstanz dort anreichert,
b) anschließend die während der Fermentation gebildete Biomasse vollständig abtrennt,
c) die so erhaltene Lösung oder Suspension durch Wasserentfernung aufkonzentriert und die Zielsubstanz ausfällt, insbesondere auskristallisieren lässt,
d) die ausgefallene bzw. auskristallisierte Substanz abtrennt,
e) die in der als Mutterlauge I bezeichneten abgetrennten Lösung enthaltene Zielsubstanz in die gewünschte Produktform überführt, während man
f) den Wassergehalt der Mutterlauge I durch Verdampfen verringert und aus der eingeengten Mutterlauge I die Zielsubstanz auskristallisieren lässt, die Kristalle abtrennt und
g) diese in den Prozeß direkt nach der vollständigen Abtrennung der Biomasse aus der eingesetzten Fermentationsbrühe wieder einschleust, und
h) die nach Abtrennung der Zielsubstanz erhaltene Mutterlauge II verwirft oder ganz oder teilweise in die Mutterlauge I zurückführt, wobei als Zielsubstanzen fermentativ hergestellte L-Aminosäuren, aufgearbeitet werden.

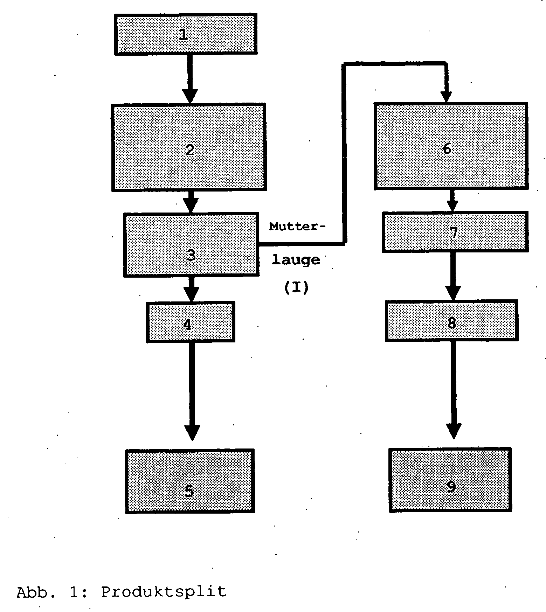

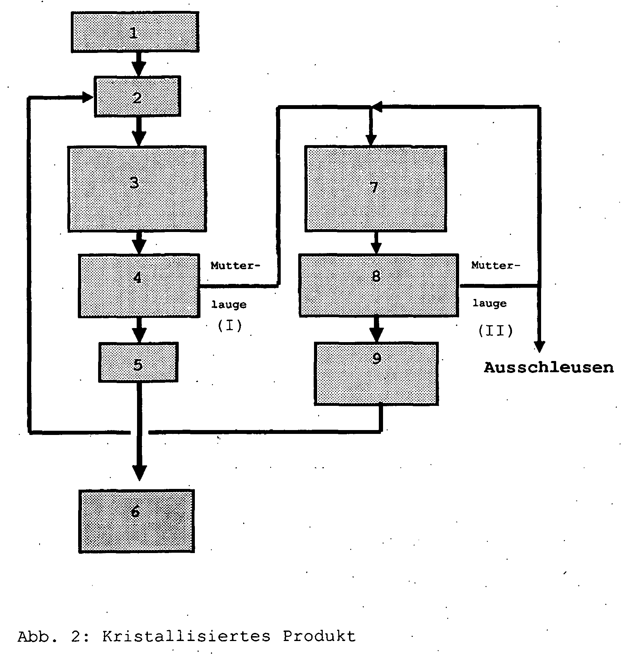

Abbildung 1 und 2 geben zwei verschiedene Varianten zur Aufarbeitung der Mutterlauge I wieder.

Von der jeweiligen Zielsubstanz bleibt in Abhängigkeit von den gegebenen Bedingungen ein gelöster Rest in der abgetrennten Mutterlauge I zurück.

Deren Konzentration kann man z. B. über die für die verschiedenen Substanzen bekannte Zusammenhänge zwischen Temperatur, Konzentration und Löslichkeitsverhalten steuern. Die in der Mutterlauge gelöste Menge an Zielsubstanz sollte so eingestellt werden, dass das aus dieser Mutterlauge I gewonnene feste Produkt einen für den Anwender attraktiven Gehalt an Zielsubstanz von 40 bis 85 Gew.-%, bevorzugt 60 bis 85 Gew.-% der Trockenmasse (getrocknet bei 80-90°C) aufweist. Daneben erhält man z. B. aus Schritt e) von Abb. 1 eine reine Zielsubstanz mit einem Gehalt von ca. 98 bis 99 Gew.-% der Trockenmasse, die man auf bekannte Art in ein Granulat oder eine sonst gewünschte Teilchenform überführt. Ein Verfahren zur Herstellung von reinen L-Threonin-Granulaten ist z. B. in der WO 2005/00675 beschrieben. Bei einem anderen Verfahren stellt man eine Suspension der reinen Kristalle her, aus der anschließend Granulate erzeugt werden.

Die abgetrennte Mutterlauge I wird in einer Variante zu einem Produkt umgearbeitet, das die bei der Abtrennung der Biomasse neben die Zielsubstanz nicht abgetrennten weiteren gelösten Bestandteile der Fermentationsbrühe enthält. Dabei wird diese Mutterlauge durch Verdampfen von Wasser eingeengt und die Zielsubstanz fällt entsprechend dem durch ihre physikalisch chemischen Eigenschaften bedingten Löslichkeitsverhalten aus. Es ist energetisch günstiger, an dieser Stelle des Verfahrens Wasser abzutrennen, um den anschließenden Granulationsprozess nicht mit nur unter hohem Aufwand abzutrennendem Wasser zu belasten (Schritte 6, 7, 8 in Abb.1).

In einer Variante wird die sich bildende Suspension naßvermahlen und granuliert. Es sind jedoch auch weitere Verfahren zur Granulation einsetzbar. Der Zielsubstanzgehalt dieses Granulats, das weitere Bestandteile der Fermentationsbrühe enthält, lässt sich auf den im Endprodukt gewünschten Wert durch Zusetzen der Zielsubstanz in fester oder gelöster Form erhöhen, wenn der Gehalt in der Mutterlauge I diesen Wert nicht erreicht. Die Zielsubstanz setzt man der Mutterlauge I bevorzugt vor deren Einengung durch Wasserverdampfung in der gewünschten Menge zu (in Schritt 6 in Abb. 1). Dabei handelt es sich bevorzugt um aus der Fest-flüssig-Trennung (Schritt 3) abgetrennten Feststoff.

In einer erfindungsgemäßen Variante wird die Mutterlauge I ebenfalls durch Verdampfen von Wasser eingeengt, die Zielsubstanz aber bevorzugt durch Kühlungskristallisation ausgefällt und von der so entstehenden Mutterlauge II abgetrennt (Schritt 8 in Abb. 2).

Diese wird entweder verworfen oder teilweise in die Mutterlauge I zurückgeführt. Dabei wird vermieden, dass sich Nebenprodukte im Kreislauf störend anreichern.

Die bei dieser Aufbereitung anfallenden Kristalle sind im allgemeinen nicht als Verkaufsprodukt geeignet. Man schleust sie in einer bevorzugten Verfahrensvariante direkt nach der Abtrennung der Biomasse aus der Fermentationsbrühe vor, während oder nach dem Einengen der dann Biomassefreien Fermentationsbrühe wieder in das Verfahren ein und erhöht so die Konzentration der Zielsubstanz in dieser Brühe. Gemäß Abb. 2 erfolgt die Rückführung in den Prozess nach Schritt 1 in Schritt 2.

Die Mengenverhältnisse der Teilströme lassen sich an folgendem Beispiel erläutern, bei dem die Herstellung einer reinen Zielsubstanz und eines Produkts, das diese Zielsubstanz zu 80 Gew.-% bezogen auf die Trockenmasse enthält, erfolgt. Nach dem Abtrennen der Biomasse gewinnt man z. B. ein Filtrat, das in 100 kg Trockenmasse 90 kg der Zielsubstanz, z. B. L-Threonin, und 10 kg feste Bestandteile aus Fermentationsbrühe enthält. Dies ist durch Analyse leicht festzustellen. Man stellt die Lösungsverhältnisse im Filtrat so ein, dass von der Zielsubstanz 50 kg auskristallisieren. Die restlichen 40 kg befinden sich dann mit den 10 kg Nebenprodukt in der Mutterlauge. Daraus lässt sich über das in Abb. 1 demonstrierte Verfahren ein 80 Gew.-%iges Trockenprodukt gewinnen. Eine derartige Aufteilung der Mengenströme, die zu Produkten mit unterschiedlichen Gehalten an Zielsubstanz führt, wird als Produktsplit bezeichnet.

Das erfindungsgemäße Verfahren ist geeignet für fermentativ herstellbare L-Aminosäuren, die sich durch Auskristallisieren oder Ausfällen aus der von der Biomasse befreiten Fermentationsbrühe abtrennen lassen, insbesondere L-Threonin, L-Homoserin, L-Tryptophan, L-Valin, L-Arginin, L-Methionin, L-Leucin, L-Isoleucin und L-Tyrosin, insbesondere aber L-Threonin. Auch fermentativ hergestelltes L-Lysin ist erfindungsgemäß aufarbeitbar. Das Verfahren ist nicht durch Genus, Familie oder Spezies der eingesetzten Mikroorganismen eingeschränkt.

Erfindungsgemäß eingesetzte, die Zielsubstanzen produzierende Mikroorganismen werden bevorzugt ausgewählt unter den Gattungen Corynebacterium, Bacillus, Escherichia, Aspergillus, Lactobacillus, insbesondere unter Stämmen von Corynebacterium glutamicum, Bacillus subtillis, Escherichia coli oder Aspergillus niger.

Die erfindungsgemäße Koppelproduktion, bei der man eine Fraktion der Zielsubstanz in reiner, bevorzugt kristalliner Form und eine weitere Fraktion erhält, die Nebenkomponenten aus der Fermentation und die Zielsubstanz enthält, ist mit folgenden Vorteilen verbunden:
- Vermeiden von mit der alleinigen Ausrichtung auf die reine Zielsubstanz verbundenen Ausbeuteverlusten durch das unzureichende Aufarbeiten der Mutterlauge.
- Vermeiden von hohen Transportkosten, die entstehen, wenn das Verfahren nur auf die Herstellung eines alle Nebenkomponenten aus der Fermentations enthaltenden Produkts ausgerichtet ist, indem für lange Vertriebswege bevorzugt die kristalline, reine Produktform aus der Koppelproduktion Verwendung findet.
- Einfache Standardisierung des Wirkstoffgehalts in dem mit Nebenkomponenten versehenen Produktanteil durch geeignete Prozesssteuerung (d. h. es ist keine Zugabe eines inerten Stoffes erforderlich).
- Reduzierte Energiekosten im Vergleich zu einem Verfahren mit Herstellung eines einzigen Produktes, welches die Nebenkomponenten aus der Fermentation enthält, da die energieintensive Trocknung und Granulation nur auf einen Teilstrom im Prozeß angewandt wird.

Erklärung zu den Abbildungen, die das Verfahren beispielhaft wiedergeben:
Abb. 1: Produktsplit
   - 1: Abtrennung der Biomasse, bevorzugt durch Ultrafiltration
   - 2: Eindampfen und Auskristallisieren, gegebenenfalls in einem Schritt
   - 3: Fest-Flüssig-Trennung (Mutterlauge I)
   - 4: Aufarbeitung der abgetrennten Kristalle, z. B. Granulation und Trocknung
   - 5: reine Zielsubstanz

   - 6: Eindampfen und Auskristallisieren aus der abgetrennten Mutterlauge I
   - 7: Aufarbeitung der anfallenden Suspension: Nassvermahlung
   - 8: Granulation
   - 9: Produkt 2: enthaltend Zielsubstanz und Nebenbestandteile aus der Fermentationsbrühe
Abb. 2: Gewinnen von kristallinem Produkt
   - 1: Abtrennung der Biomasse durch Ultrafiltration
   - 2: Einbringen der Kristalle aus 9
   - 3: Eindampfen und Auskristallisieren, gegebenenfalls in einem Schritt
   - 4: Fest- Flüssig-Trennung (Mutterlauge I)

   - 5: Aufarbeitung der abgetrennten Kristalle, z. B. durch Granulation und Trocknung
   - 6: Produkt 1: reine Zielsubstanz
   - 7: Eindampfen und Auskristallisieren aus der Mutterlauge (I)
   - 8: Gegebenenfalls auch Auskristallisieren durch Kühlkristallisation
   - 9: Fest-Flüssig-Trennung
   - 10: Rückführung eines Teilstroms der dabei gebildeten Mutterlauge II nach 7

## Patentansprüche

1. Verfahren zur Herstellung von Produkten, die mindestens eine fermentativ hergestellte Zielsubstanz enthalten in mehreren Verfahrensstufen, bei denen man
a) die die gewünschte Zielsubstanz produzierenden Mikroorganismen in einem geeigneten Medium fermentiert und die Zielsubstanz dort anreichert,
b) anschließend die während der Fermentation gebildete Biomasse vollständig abtrennt,
c) die so erhaltene Lösung oder Suspension durch Wasserentfernung aufkonzentriert und die Zielsubstanz ausfällt, insbesondere auskristallisieren lässt,
d) die ausgefallene bzw. auskristallisierte Substanz abtrennt,
e) die in der als Mutterlauge I bezeichneten abgetrennten Lösung enthaltene Zielsubstanz in die gewünschte Produktform überführt, während man
f) den Wassergehalt der Mutterlauge I durch Verdampfen verringert und aus der eingeengten Mutterlauge I die Zielsubstanz auskristallisieren lässt, die Kristalle abtrennt und
g) diese in den Prozeß direkt nach der vollständigen Abtrennung der Biomasse aus der eingesetzten Fermentationsbrühe wieder einschleust, und
h) die nach Abtrennung der Zielsubstanz erhaltene Mutterlauge II verwirft oder ganz oder teilweise in die Mutterlauge I zurückführt, wobei als Zielsubstanzen fermentativ hergestellte L-Aminosäuren, aufgearbeitet werden.

2. Verfahren gemäß Anspruch 1, bei dem man der Mutterlauge I reine Zielsubstanz zusetzt.

3. Verfahren gemäß Ansprüchen 1 oder 2, bei dem es sich bei der Zielsubstanz um Verbindungen, ausgewählt aus der Gruppe L-Threonin, L-Homoserin, L-Tryptophan, L-Valin, L-Arginin, L-Methionin, L-Leucin, L-Isoleucin und L-Tyrosin handelt.

4. Verfahren gemäß Anspruch 1, bei dem man
a) L-Threonin fermentativ gemäß Anspruch 1 a) bis 1 d) herstellt und abtrennt,
b) das abgetrennte L-Threonin granuliert und ein Granulat mit einem L-Threoningehalt von ca. 98 bis 99 Gew.-% gewinnt.

## Claims

1. Method for producing products which comprise at least one target substance produced by fermentation in a plurality of stages, in which
a) the microorganisms producing the desired target substance are fermented in a suitable medium and the target substance is accumulated therein,
b) then the biomass formed during the fermentation is separated off completely,
c) the solution or suspension obtained in this way is concentrated by removing the water, and the target substance is precipitated out, in particular left to crystallize out,
d) the precipitated-out or crystallized-out substance is separated off,
e) the target substance present in the separated-off solution referred to as mother liquor I is converted to the desired product form while
f) the water content of the mother liquor I is reduced by evaporation and the target substance is left to crystallize out of the concentrated mother liquor I, the crystals are separated off and
g) these are introduced again into the process directly after completely separating off the biomass from the fermentation broth used, and
h) the mother liquor II obtained after separating off the target substance is discarded or completely or partly returned to the mother liquor I, wherein L-amino acids produced by fermentation are worked up as the target substances.

2. Method according to Claim 1, in which pure target substance is added to the mother liquor I.

3. Method according to Claims 1 or 2, in which the target substance is compounds selected from the group consisting of L-threonine, L-homoserine, L-tryptophan, L-valine, L-arginine, L-methionine, L-leucine, L-isoleucine and L-tyrosine.

4. Method according to Claim 1, in which
a) L-threonine is produced by fermentation according to Claim 1 a) to 1 d) and is separated off,
b) the separated-off L-threonine is granulated and granules with an L-threonine content of ca. 98 to 99% by weight are obtained.

## Revendications

1. Procédé de fabrication de produits qui contiennent au moins une substance cible fabriquée par fermentation en plusieurs étapes de procédé, selon lequel
a) les microorganismes produisant la substance cible souhaitée sont fermentés dans un milieu approprié et la substance cible y est concentrée, puis
b) la biomasse formée pendant la fermentation est séparé en totalité,
c) la solution ou suspension ainsi obtenue est concentrée par élimination d'eau et la substance cible est précipitée, notamment laissée cristalliser,
d) la substance précipitée ou cristallisée est séparée,
e) la substance cible contenue dans la solution séparée nommée liqueur mère I est transformée en la forme de produit souhaitée, tandis que
f) la teneur en eau de la liqueur mère I est réduite par évaporation et la substance cible est laissée cristalliser à partir de la liqueur mère I concentrée, les cristaux sont séparés, et
g) ceux-ci sont réintroduits dans le procédé directement après la séparation totale de la biomasse du bouillon de fermentation utilisé, et
h) la liqueur mère II obtenue après la séparation de la substance cible est mise au rebut ou recyclée en totalité ou en partie dans la liqueur mère I, des acides L-aminés fabriqués par fermentation étant traités en tant que substances cibles.

2. Procédé selon la revendication 1, selon lequel la substance cible pure est ajoutée à la liqueur mère I.

3. Procédé selon les revendications 1 ou 2, selon lequel la substance cible consiste en des composés choisis dans le groupe constitué par la L-thréonine, la L-homosérine, le L-tryptophane, la L-valine, la L-arginine, la L-méthionine, la L-leucine, la L-isoleucine et la L-tyrosine.

4. Procédé selon la revendication 1, selon lequel
a) de la L-thréonine est fabriquée par fermentation selon la revendication 1a) à 1d) et séparée,
b) la L-thréonine séparée est granulée et un granulat ayant une teneur en L-thréonine d'environ 98 à 99 % en poids est obtenu.
